(19) **Europäisches Patentamt**
European Patent Office
Office européen des brevets

(11) **EP 1 444 268 B1**

(12) **EUROPÄISCHE PATENTSCHRIFT**

(45) Veröffentlichungstag und Bekanntmachung des Hinweises auf die Patenterteilung:
**13.06.2007 Patentblatt 2007/24**

(51) Int Cl.:
***C07K 16/46*** (2006.01)

(21) Anmeldenummer: **02779543.4**

(22) Anmeldetag: **09.11.2002**

(86) Internationale Anmeldenummer:
**PCT/EP2002/012545**

(87) Internationale Veröffentlichungsnummer:
**WO 2003/042231 (22.05.2003 Gazette 2003/21)**

(54) **BISPEZIFISCHES ANTI-CD28 ANTIKÖRPER-MOLEKÜL**

BISPECIFIC ANTI-CD28 ANTIBODY MOLECULE

MOLECULE D'ANTICORPS ANTI-CD28 BISPECIFIQUE

(84) Benannte Vertragsstaaten:
**AT BE BG CH CY CZ DE DK EE ES FI FR GB GR IE IT LI LU MC NL PT SE SK TR**

(30) Priorität: **12.11.2001 DE 10156482**

(43) Veröffentlichungstag der Anmeldung:
**11.08.2004 Patentblatt 2004/33**

(73) Patentinhaber: **Jung, Gundram**
**72108 Rottenburg-Wendelsheim (DE)**

(72) Erfinder: **Jung, Gundram**
**72108 Rottenburg-Wendelsheim (DE)**

(74) Vertreter: **Viering, Jentschura & Partner**
**Postfach 22 14 43**
**80504 München (DE)**

(56) Entgegenhaltungen:
**EP-A- 0 440 373      WO-A-94/13806**
**WO-A-97/47271**

- **SCHOONJANS R ET AL: "Fab chains as an efficient heterodimerization scaffold for the production of recombinant bispecific and trispecific antibody derivatives" JOURNAL OF IMMUNOLOGY, WILLIAMS & WILKINS CO, US, Bd. 165, Nr. 12, 15. Dezember 2000 (2000-12-15), Seiten 7050-7057, XP002207879 ISSN: 0022-1767 in der Anmeldung erwähnt**
- **BODE C ET AL: "ANTIBODY-DIRECTED FIBRINOLYSIS AN ANTIBODY SPECIFIC FOR BOTH FIBRIN AND TISSUE PLASMINOGEN ACTIVATOR" JOURNAL OF BIOLOGICAL CHEMISTRY, Bd. 264, Nr. 2, 1989, Seiten 944-948, XP001154126 ISSN: 0021-9258**
- **DATABASE EMBL [Online] ACCESSION NO: AJ507107, 2. September 2002 (2002-09-02) GROSSE-HOVEST L.: "Synthetic construct for anti-CD28 and anti-HMWG scFv antibody, clone r28M" XP002250207**
- **GROSSE-HOVEST LUDGER ET AL: "A recombinant bispecific single-chain antibody induces targeted, supra-agonistic CD28-stimulation and tumor cell killing." EUROPEAN JOURNAL OF IMMUNOLOGY, Bd. 33, Nr. 5, 20. Mai 2003 (2003-05-20), Seiten 1334-1340, XP002250206 ISSN: 0014-2980**

Printed by Jouve, 75001 PARIS (FR)

**EP 1 444 268 B1**

SEQUENZPROTOKOLL

<110> Jung, Gundram

<120> Bispezifisches Antikörper-Molekül

<130> 2812P101WO

<140> PCT/EP 02/12545
<141> 2002-11-09

<150> 101 56 482.1-44 DE
<151> 2001-11-12

<160> 3

<170> PatentIn version 3.1

<210> 1
<211> 15
<212> PRT
<213> Künstliche Sequenz

<220>
<223> Flexibler Linker FL zwischen den variablen Ketten VL und VH jeweils einer Spezifität

<400> 1

```
Gly Gly Gly Gly Ser Gly Gly Gly Gly Ser Gly Gly Gly Gly Ser
1               5                   10                  15
```

<210> 2
<211> 19
<212> PRT
<213> Künstliche Sequenz

<220>
<223> Linker L zwischen den beiden variablen Ketten VH der Spezifitäten

<400> 2

```
Ala Ser Thr Lys Gly Pro Ser Val Phe Pro Leu Ala Pro Ser Ser Ser
1               5                   10                  15

Gly Ser Gly
```

<210> 3
<211> 543
<212> PRT
<213> Künstliche Sequenz

<220>
<223> Bispezifisches Antikörpermolekül mit der Orientierung: $(V_L - FL- V_H)_{9.3} - L- (V_H- FL- V_L)_{9.2.27}$ -6his

<400> 3

Met Glu Thr Asp Thr Leu Leu Leu Trp Val Leu Leu Leu Trp Val Pro
1               5                    10                  15

Gly Ser Thr Gly Asp Ile Val Leu Thr Gln Ser Pro Ala Ser Leu Ala
                20                  25              30

Val Ser Leu Gly Gln Arg Ala Thr Ile Ser Cys Arg Ala Ser Glu Ser
        35                  40                  45

Val Glu Tyr Tyr Val Thr Ser Leu Met Gln Trp Tyr Gln Gln Lys Pro
        50                  55                  60

Gly Gln Pro Pro Lys Leu Leu Ile Phe Ala Ala Ser Asn Val Glu Ser
65                  70                  75                  80

Gly Val Pro Ala Arg Phe Ser Gly Ser Gly Ser Gly Thr Asn Phe Ser
                85                  90                  95

Leu Asn Ile His Pro Val Asp Glu Asp Asp Val Ala Met Tyr Phe Cys
            100                 105                 110

Gln Gln Ser Arg Lys Val Pro Tyr Thr Phe Gly Gly Gly Thr Lys Leu
            115                 120                 125

Glu Ile Lys Arg Gly Gly Gly Gly Ser Gly Gly Gly Gly Ser Gly Gly
        130                 135                 140

Gly Gly Ser Gln Val Lys Leu Gln Gln Ser Gly Pro Gly Leu Val Thr
145             150             155             160

Pro Ser Gln Ser Leu Ser Ile Thr Cys Thr Val Ser Gly Phe Ser Leu
                165             170             175

Ser Asp Tyr Gly Val His Trp Val Arg Gln Ser Pro Gly Gln Gly Leu
                180             185             190

Glu Trp Leu Gly Val Ile Trp Ala Gly Gly Gly Thr Asn Tyr Asn Ser
            195             200             205

Ala Leu Met Ser Arg Lys Ser Ile Ser Lys Asp Asn Ser Lys Ser Gln
    210             215             220

Val Phe Leu Lys Met Asn Ser Leu Gln Ala Asp Asp Thr Ala Val Tyr
225             230             235             240

Tyr Cys Ala Arg Asp Lys Gly Tyr Ser Tyr Tyr Tyr Ser Met Asp Tyr
                245             250             255

Trp Gly Gln Gly Thr Thr Val Thr Val Ser Ser Ala Ser Thr Lys Gly
                260             265             270

Pro Ser Val Phe Pro Leu Ala Pro Ser Ser Ser Gly Ser Gly Gln Val
        275             280             285

Lys Leu Gln Gln Ser Gly Pro Glu Leu Val Lys Pro Gly Ala Ser Val
        290             295             300

Lys Ile Ser Cys Lys Ala Ser Gly Tyr Ala Phe Ser Arg Ser Trp Met
305             310             315             320

Asn Trp Val Lys Gln Arg Pro Gly Gln Gly Leu Glu Trp Ile Gly Arg
                325             330             335

Ile Tyr Pro Gly Asp Gly Asp Thr Asn Tyr Asn Gly Lys Phe Lys Gly
            340             345             350

Lys Ala Thr Leu Thr Ala Asp Lys Ser Ser Ser Thr Ala Tyr Met Gln
        355             360             365

Val Ser Ser Leu Thr Ser Val Asp Ser Ala Val Tyr Phe Cys Ala Arg
        370             375             380

Gly Asn Thr Val Val Val Pro Tyr Thr Met Asp Tyr Trp Gly Gln Gly
385             390             395             400

Thr Thr Val Thr Val Ser Ser Gly Gly Gly Gly Ser Gly Gly Gly Gly
                405             410             415

Ser Gly Gly Gly Gly Ser Asp Ile Glu Leu Thr Gln Ser Pro Ala Ser
            420             425             430

Leu Ala Val Ser Leu Gly Gln Arg Ala Thr Ile Ser Cys Arg Ala Ser
            435             440             445

Glu Ser Val Asp Ser Tyr Gly Asn Ser Phe Met His Trp Tyr Gln Gln
        450             455             460

Lys Pro Gly Gln Pro Pro Lys Leu Leu Ile Tyr Leu Ala Ser Asn Leu
465             470             475             480

Glu Ser Gly Val Pro Ala Arg Phe Ser Gly Ser Gly Ser Arg Thr Asp
            485             490             495

Phe Thr Leu Thr Ile Asp Pro Val Glu Ala Asp Asp Ala Ala Thr Tyr
            500             505             510

Tyr Cys Gln Gln Asn Asn Glu Asp Pro Leu Thr Phe Gly Gly Gly Thr
        515             520             525

Lys Leu Glu Leu Lys Arg Ala Ala Ala His His His His His His
        530             535             540

14

## Beschreibung

Die vorliegende Erfindung betrifft ein bispezifisches Antikörper-Molekül, ein bispezifisches Antikörper-Molekül mit Bivalenz für CD28, für das Antikörper-Molekül kodierende Nukleinsäuren, das Antikörper-Molekül und/oder die für das Antikörper-Molekül kodierende Nukleinsäure enthaltende Zellen und die Verwendung des bispezifischen Antikörper-Moleküls.

Natürliche Antikörper sind im einfachsten Fall Y-förmige, tetramere Glykoproteine. Ihre typische Struktur besteht aus zwei identischen leichten Ketten und zwei identischen schweren Ketten. Die leichte Kette setzt sich aus den zwei Domänen $V_L$ und $C_L$ zusammen, die schwere Kette dagegen aus den vier Domänen $V_H$, $C_H1$, $C_H2$ und $C_H3$. Dabei kennzeichnen die Buchstaben C und V jeweils die konstanten und die variablen Teile des Antikörpers. Disulfid-Brücken verbinden die beiden schweren Ketten untereinander, außerdem die schweren mit den leichten Ketten.

Als "rekombinante Antikörper" werden im allgemeinen Antigenbindende Fragmente eines Antikörpers bezeichnet, welcher von einer heterologen Quelle produziert wird. Die kleinste Antikörper-Einheit, die noch ein Antigen binden kann, sind $F_v$-Fragmente, welche die variable Domäne der schweren und der leichten Kette enthalten.

In Forschung und Therapie werden häufig die sogenannten single-chain $F_v$-Fragmente (scF$_v$) verwendet. In diesen scF$_v$-Fragmenten ist die variable Domäne der schweren Kette mit der variablen Domäne der leichten Kette durch einen kurzen Peptidspacer kovalent verbunden. Dieser Spacer wird auf der genetischen Ebene eingeführt. Die scF$_v$-Fragmente können durch das Hinzufügen kurzer Markierungssequenzen entweder am N-Terminus oder am C-Terminus gereinigt und detektiert werden.

Unter den rekombinanten Antikörpern spielen die bispezifischen Antikörper eine große Rolle, bei denen Antigen-Bindestellen gegen zwei unterschiedliche Antigene miteinander verbunden sind. Verschiedene Strategien stehen zur Verfügung, um bispezifische rekombinante Antikörperfragmente herzustellen. So können bspw. zwei unterschiedliche scF$_v$-Fragmente durch die Einführung eines zusätzlichen Linkers zwischen dem C-Terminus des ersten scF$_v$-Fragments und dem N-Terminus des zweiten scF$_v$-Fragments verbunden werden. Bei der Verwendung zweier identischer scF$_v$-Fragmente erhält man Antikörper, die in bezug auf eine Bindestelle bivalent sind, bei Verwendung zweier unterschiedlicher scF$_v$-Fragmente generiert man Fragmente, die in bezug auf eine Bindestelle monovalent sind, gleichzeitig aber bispezifisch, da sie zwei unterschiedliche Bindestellen aufweisen.

Um sogenannte Diabodies herzustellen, wird ein sehr kurzer Linker zwischen der variablen Domäne der schweren Kette und der variablen Domäne der leichten Kette gewählt, um so das Verbinden der $V_H$- und $V_L$-Domänen einer Kette zu verhindern. Dadurch können dimere Moleküle gebildet werden, bei denen die $V_H$- und $V_L$-Domänen zweier unterschiedlicher Ketten ein doppelköpfiges Molekül bilden. Bei der Verwendung von zwei unterschiedlichen, nicht gekoppelten Antikörperspezifitäten (bspw. A und B), die in der Reihenfolge $V_{HA}$-$V_{LB}$ und $V_{HB}$-$V_{LA}$ in der gleichen Zelle exprimiert werden, kann man bispezifische Diabodies bilden. Diese dimeren Diabody-Moleküle können auch durch ein monomeres Molekül hergestellt werden, wenn man die zwei $V_H$-$V_L$-Fragmente mit einem zusätzlichen Peptidlinker covalent verbindet (single-chain Diabody, scDb). Solche dimeren bispezifischen Antikörper besitzen also zwei Valenzen für jede Spezifität.

Bispezifische Diabodies oder Antikörper wurden vor allem deswegen generiert, um sowohl die Valenz als auch die Stabilität und damit das therapeutische Potential zu steigern. Bisher konnte gezeigt werden, daß durch single-chain Diabodies vor allem die Bindungsstärke verbessert werden konnte.

Tomlinson und Holliger, "Methods for Generating Multivalent and Bispecific Antibody Fragments", Methods in Enzymology (2000) 326:461-479, beschreiben die Herstellung von bispezifischen Diabodies. Solche bispezifischen Diabodies erwiesen sich als effektiv in der Rekrutierung der cytotoxischen T-Zellen, des Komplements und der Antikörper-abhängigen Effekter-Funktionen, wie bspw. Zell-vermittelte Cytotoxizität und Phagocytose.

Kipriyanov et al., "Bispecific Tandem Diabody for Tumor Therapy with improved Antigen Binding and Pharmacokinetics" J. Mol. Biol. (1999) 293:41-56, beschreiben eine Diabody-Bildung eines single-chain-Moleküles mit vier V-Regionen zweier unterschiedlicher Spezifitäten. Gleichzeitig beschreibt die Arbeitsgruppe eine Dimerbildung dieses single-chain Antikörpers bzw. einen bispezifischen Tandemdiabody. Die Tandemdiabodies sind also bivalent für jede Spezifität, in der vorliegenden Veröffentlichung für CD19 und CD3. Die Tandem-Diabodies zeigten zwar eine gesteigerte Affinität zu ihrem Antigen, eine damit verbundene gesteigerte biologische Aktivität konnte aber nicht nachgewiesen werden. So besaßen diese Tandem-Diabodies eine nur gering erhöhte Aktivität bezüglich der Abtötung von Tumorzellen im Vergleich zu den single-chain-Diabodies.

Damit naive T-Zellen an einer anpassungsfähigen Immunantwort teilnehmen können, müssen sie zur Proliferation und Differenzierung aktiviert werden. Diese Aktivierung von naiven T-Zellen erfolgt durch die Erkennung eines fremden Peptidfragments mit dem Antigen-spezifischen T-Zell Rezeptor CD3-Komplex. Generell ist anerkannt, daß die effektive Aktivierung von naiven T-Zellen zur Proliferation und Differenzierung ein zweites oder costimulierendes Signal zusätzlich zu dem Antigen-spezifischen Stimulus über den TCR/CD3-Komplex benötigt. Die am besten charakterisierten costimulatorischen Moleküle auf Antigenpräsentierenden Zellen sind die Glykoproteine B7.1 und B7.2.

Der Rezeptor für B7-Moleküle auf T-Zellen ist CD28, und die Ligation von CD28 durch B7 Moleküle (oder durch anti-CD28 Antikörper) costimuliert das Wachstum von naiven T-Zellen. Nach der Aktivierung der T-Zellen wird auch die Expression von CD28 gesteigert.

Zwischenzeitlich konnten noch weitere costimulierende Rezeptoren identifiziert werden, dennoch ist bisher die B7/CD28 Interaktion der weitaus stärkste Costimulus für naive T-Zellen.

Auf naiven, ruhenden T-Zellen ist CD28 der einzige Rezeptor für B7 Moleküle. Sobald aber die Zellen aktiviert werden, exprimieren sie den zusätzlichen Rezeptor CTLA-4. CTLA-4 bindet B7-Moleküle weitaus stärker als CD28 und liefert negative Signale an die aktivierte T-Zelle, wodurch u.a. weniger Interleukin-2 gebildet wird. Deshalb erwies sich ein therapeutischer Einsatz von B7-Proteinen als costimulierende Moleküle als zweifelhaft, da der auf durch B7 aktivierte T-Zellen exprimierte CTLA-4-Rezeptor die T-Zell-Aktivierung unterdrückt.

Eine Stimulierung des TCR/CD3-Komplexes mit bispezifischen Antikörpern induzierte bei systemischen in vivo-Anwendungen beim Menschen schwerwiegende und unerwartete Nebenwirkungen (Tibben et al.: "Pharmacogenetics, biodistribution and biological effects of intravenously administered bispecific monoclonal antibody OC/TR Fab2 in ovarian carcinoma patients", (1996) Int. J. Cancer 66:447-483).

Vor einigen Jahren wurde in verschiedenen Veröffentlichungen gezeigt, daß chemisch hybridisierte bispezifische Antikörper gerichtet gegen Tumor-assoziierte Antigene und gegen CD28 eine T-Zell-Costimulierung gerichtet gegen Tumorzellen in vitro und *in vivo* induzieren: siehe z.B. Jung, G. und Müller-Eberhard, H.J.: "An in vitro model for tumor immunotherapy with antibody heteroconjugates" Immunology Today (1988) 9:257-260; Große-Hovest, L. et al.: "Tumor growth inhibition with bispecific antibody fragments in a syngeneic mouse melanoma model: The role of targeted T cell costimulation via CD28" Int. J. Cancer (1999) 80:138-144.

In den Experimenten wurden diese bispezifischen Antikörper in Kombination mit bispezifischen Antikörpern, die den TCR/CD3-Komplex stimulieren, verwendet. Alleine verwendet, waren sie nur gering effektiv.

1996 konnten Hayden et al., "Costimulation by CD28sFv expressed on the tumor cell surface or as a soluble bispecific molecule targeted to the L6 carcinoma antigen" Tissue Antigen (1996) 48:242-254, zeigen, daß transfizierte CD28 single-chain Antikörper und ein rekombinanter bispezifischer Antikörper mit Tumor/CD28-Spezifität ähnliche costimulatorische Effekte in T-Zell-Blasten hervorrufen konnten, wobei die Blasten durch Phythämagglutinin bereits zur Proliferation stimuliert waren. Das beschriebene single-chain scFv-Molekül ist jedoch nicht supra-agonistisch.

Vor diesem Hintergrund liegt der vorliegenden Anmeldung die Aufgabe zugrunde, ein Reagenz bereitzustellen, das T-Zellen supra-agonistisch, also ohne ein zusätzliches costimulierendes Signal effektiv stimulieren kann.

Diese Aufgabe wird erfindungsgemäß gelöst durch ein bispezifisches Antikörper-Molekül gemäss Anspruch 1.

Das bispezifische Antikörper-Molekül mit Spezifität für den T-Zell-Rezeptor CD28 und Spezifität für ein Tumor-Antigen ist bivalent für CD28.

Insgesamt ist bevorzugt, wenn die Bindestelle für den T-Zell-Rezeptor CD28 an den humanen T-Zell-Rezeptor CD28 bindet. Dessen Sequenz ist bspw. veröffentlicht in Aruffo A. et al., Proc. Natl. Acad. Sci. U.S.A. 84:8573-8577 (1987).

Die Aufgabe wird außerdem durch ein Verfahren zur Behandlung von Zellen gelöst, bei dem das erfindungsgemäße bispezifische Antikörper-Molekül eingesetzt wird, um eine supra-agonistische tumorzellinduzierte Aktivierung von T-Zellen zu bewirken, so daß keine zusätzlichen exogenen Stimuli benötigt werden.

Die der Erfindung zugrunde liegende Aufgabe wird auf diese Weise vollkommen gelöst.

Die Erfinder der vorliegenden Anmeldung konnten nämlich zeigen, daß es möglich ist, durch alleinige Aktivierung des costimulatorischen Rezeptors CD28 mittels eines erfindungsgemäßen bispezifischen Antikörpermoleküls oder mittels eines Dimers des ersten erfindungsgemäßen bispezifischen Antikörper-Moleküls T-Zellen effektiv zu stimulieren, ohne daß ein weiterer Antigenspezifischer Stimulus über den TCR/CD3-Komplex notwendig ist. Das bedeutet also z.B., daß dieser Prozeß in gerichteter Weise auf der Oberfläche von Tumorzellen erfolgt.

Gleichzeitig wird vermieden, daß es zur Stimulation von CTLA-4-Molekülen auf den aktivierten T-Zellen kommt. Durch Vermeidung der Stimulation dieser supprimierenden Moleküle wird die Proliferation der T-Zellen nicht begrenzt, wodurch eine angemessene Immunantwort nicht unterdrückt wird.

Mit anderen Worten, die T-Zellen werden aktiviert, wenn das bispezifische Antikörper-Molekül das CD28-Molekül auf den T-Zellen bivalent bindet und gleichzeitig mit seiner anderen Bindungsstelle an das Tumor-Antigen auf einer Tumorzelle bindet. Es handelt sich dabei sozusagen um eine tumorzellinduzierte, supra-agonistische Aktivierung von T-Zellen, bei der das erfindungsgemäße bispezifische Antikörper-Molekül dazu in der Lage ist, die T-Zellen ohne zusätzliches Signal effektiv zu stimulieren, wenn es neben dem CD 28-Molekül auch an das Tumor-Antigen gebunden hat. Es handelt sich damit um eine selektive, tumorzellinduzierte Aktivierung von T-Zellen, bei der keine Bindung an den TCR/CD3-Komplex erforderlich ist.

Die Erfinder der vorliegenden Anmeldung haben in verschiedenen eigenen Versuchen weiterhin gezeigt, daß die bispezifischen Antikörper-Holeküle in Bezug auf die Abtötung von Tumorzellen ausgesprochen effektiv waren.

Die Erfinder haben gefunden, daß das Antikörper-Molekül bei rekombinanter Herstellung dazu neigt, spontan Dimere zu bilden, und daß die Dimere wegen ihrer Bivalenz für CD28 besonders effizient T-Zellen stimulieren. Die Dimere können dabei auch gezielt erzeugt werden. Da die Bivalenz für CD28 die supraagonistischen Eigenschaften des neuen Antikörper-Moleküls bewirken, zeigt auch das zweite Antikörper-Molekül diese supraagonistische Wirkung, denn es ist ebenfalls bivalent für CD28, während es für das Tumor-Antigen mono- oder bivalent sein kann.

In einem Ausführungsbeispiel ist das bispezifische Antikörper-Molekül daher mit einem weiteren bispezifischen Antikörper-Molekül zu einem Dimer verbunden.

Anhand von eigenen Versuchen haben die Erfinder gezeigt, daß erfindungsgemäße bispezifische Antikörper-Moleküle überraschenderweise dimerisieren. Eine Dimerisierung derartig konstruierter Antikörper-Moleküle war bisher nicht beschrieben und deswegen auch völlig unerwartet.

Durch die Dimerisierung weist das bispezifische Antikörper-Molekül zwei Bindestellen für CD28 und zwei Bindestellen für das Tumor-Antigen auf. Die Erfinder konnten mit einem derartigen Antikörper-Molekül eine effektive tumorzellinduzierte Aktivierung von T-Zellen zeigen, was zu einer effizienten Abtötung von Tumor-Zellen führte, die das Tumor-Antigen exprimierten.

Das Antikörper-Molekül kann weiterhin an eine der leichten Ketten zumindest ein Teil eines fos-jun-Adaptors oder einer Hinge-Region fusioniert sein.

Durch Anfusionieren eines fos-jun-Adaptors kann eine Dimerisierung gezielt herbeigeführt werden, da die Genprodukte des fos-jun-Adaptors spontan assoziieren.

Unter einer Hinge-Region wird der Abschnitt der schweren Kette eines Immunglobulins verstanden, der zwischen der ersten und zweiten konstanten Domäne liegt. Auch diese Maßnahme führt zu einer gezielten Dimerbildung.

Eine Verwendung dieser funktionellen Einheiten und Beispiele für deren Sequenzen sind z.B. zusammengefaßt von van Spriel et al.: "Immunotherapeutic Perspective for Bispecific Antibodies" Immunol. Today (2000) 21(8):391-397.

Allgemein ist bevorzugt, wenn das Tumor-Antigen ausgewählt ist aus der Gruppe umfassend Melanom-assoziiertes Proteoglykan, HER-2/neu oder CD20.

HER-2/neu ist ein Oncogen und spielt v.a. bei Brustkrebs eine große Rolle. CD20 ist ein Antigen, das insbesondere auf Tumor-Zellen vom B-Zell-Typ gefunden wird.

Die Aufzählung dieser Tumor-Antigene ist lediglich beispielhaft, die Erfindung bezieht auch andere Zelloberflächenmoleküle mit ein, die Tumor-assoziiert sind.

Weiterhin ist bevorzugt, wenn der Peptidlinker bei dem ersten Antikörper-Molekül zumindest einen Teil des N-Terminus der $C_H1$ Domäne des menschlichen IgG aufweist.

Dabei ist insbesondere bevorzugt, wenn der Teil des N-Terminus der $C_H1$ Domäne des menschlichen IgGs die Aminosäuresequenz Ala-Ser-Thr-Lys-Gly-Pro-Ser-Val-Phe-Pro-Leu-Ala-Pro-Ser-Ser-Ser-Gly-Ser-Gly enthält.

An die beiden konstanten Domänen eines Fab-Fragments mit Anti-Tumor-Spezifität ist jeweils ein scFv-Fragment mit CD28-Spezifiät fusioniert.

Durch diese Konstruktion wird ein Antikörper-Molekül geschaffen, das für CD28 bivalent und für das Tumor-Antigen monovalent ist.

Eine Konstruktion dieses Typs aber mit anderen Spezifitäten wird bspw. beschrieben von Schoonjans, R. et al.: "Fab Chains as an Efficient Heterodimerization Scaffold for the Production of Recombinant Bispecific and Tri-specific Antibody Derivatives", J. Immunol. (2000) 165(12):7050-7057.

Desweiteren ist das bispezifische Antikörper-Molekül auch für das Tumor-Antigen bivalent.

Das erfindungsgemäße bispezifische Antikörper-Molekül ist bivalent für CD28 und mono- oder bivalent für das Tumor-Antigen, wobei durch die Bivalenz für CD28 die erwähnte supraagonistische Wirkung erzielt wird, wodurch die T-Zellen ohne zusätzliches Signal stimuliert werden, aber nur dann, wenn neben der bivalenten Bindung an das CD28-Molekül auf der T-Zelle auch eine mono- oder bivalente Bindung an das Tumor-Antigen auf der Tumorzelle erfolgt ist.

Desweiteren ist ein Antiköpermolekül beschrieben an dessen schweren Ketten eines kompletten Antikörpers mit Anti-Tumor-Spezifität jeweils ein ScFv-Fragment mit CD28-Spezifität fusioniert ist.

Unter einem "kompletten" Antikörper wird dabei ein Antikörper verstanden, der die Struktur eines Immunglobulins besitzt. Diese enthalten zwei schwere Ketten mit jeweils einer variablen und drei konstanten Domänen und zwei leichte Ketten mit jeweils einer variablen und einer konstanten Domäne.

Durch die Fusionierung jeweils eines scFv-Fragments mit CD28-Spezifität an ein solches komplettes Antikörper-Molekül mit Anti-Tumor-Spezifität wird ein bispezifisches Antikörper-Molekül geschaffen, das sowohl für CD28 als auch für ein Tumor-Antigen bivalent ist.

Eine Konstruktion dieses Typs aber mit anderen Spezifitäten und deren Funktionalität ist bspw. beschrieben in van Spriel et al.: "Immunotherapeutic Perspective for Bispecific Antibodies", Immunol. Today (2000) 21(8):391-397.

Die Erfindung betrifft außerdem eine Nukleinsäure, kodierend für ein erfindungsgemäßes, bispezifisches Antikörper-Molekül. Die Nukleinsäure ist im allgemeinen eine DNA oder eine RNA, vorzugsweise doppelsträngige DNA.

Gegenstand der Erfindung ist weiterhin ein Vektor, der die erfindungsgemäße Nukleinsäure umfaßt. Der Vektor kann hierbei ein viraler oder ein nicht-viraler Vektor sein.

In einer Ausführungsform der Erfindung wird die Nukleinsäure oder der Vektor in einer Zelle exprimiert. Die Zelle kann dabei aus der Gruppe umfassend Säuger-, Bakterien-, Insekten, Pflanzen- oder Hefezellen unfassen. Die mit der erfindungsgemäßen Nukleinsäure oder dem erfindungsbemäßen Vektor transformierte oder transfizierte Zelle wird kultiviert und das exprimierte Genprodukt anschließend isoliert.

Ein weiterer Gegenstand der Erfindung ist eine pharmazeutische Zusammensetzung mit einem erfindungsgemäßen bispezifischen Antikörper-Molekül und einem pharmazeutisch akzeptierbaren Träger.

Das erfindungsgemäße bispezifische Antikörper-Molekül kann dabei zur tumorzellinduzierten T-Zell-Aktivierung verwendet werden, insbesondere bei der Therapie und/oder Prophylaxe von Tumorerkrankungen, um solche Tumorzellen selektiv über die Aktivierung der T-Zellen abzutöten, die das Tumor-Antigen exprimieren. Eine selektive Abtötung von Tumorzellen kann bspw. in vitro dadurch erfolgen, daß T-Zellen und das Tumor-Antigen exprimierende Tumorzellen zusammen mit der erfindungsgemäßen bispezifischen Antikörper-Molekül inkubiert werden. *In vivo* kann ein selektives Abtöten von Tumorzellen durch die Verabreichung eines Medikamentes erfolgen, das das bispezifische Antikörper-Molekül enthält. Dieses Molekül bindet sich dann einerseits mit seiner Bindungsstelle für das Tumor-Antigen an die so definierte Tumorzelle und andererseits an im Körper vorhandene T-Zellen, und zwar über die bivalente Bindungsstelle für das CD28-Molekül, das auf den T-Zellen exprimiert ist. Da eine weitere Stimulierung der T-Zellen über den TCR/CD3-Komplex nicht erforderlich ist, kommt es auf diese supra-agonistische Weise zu einer tumorzellinduzierten Aktivierung der T-Zellen und damit zu einem selektiven Abtöten der Tumorzellen.

Bei menschlichen T-Zellen konnte zwar eine TCR/CD3-unabhängige Aktivierung bei Verwendung eines besonders immobilisierten CD28-Antikörpers (BW828) beobachtet werden, diese erwies sich jedoch als nur gemäßigt, und der auch in Ausführungsbeispielen der vorliegenden Anmeldung verwendete 9.3 (CD28)-Antikörper zeigte sich als nur äußerst gering effektiv in der Aktivierung von T-Zellen: siehe Siefken et al.: "A CD28 associated signaling pathway leading to cytokine gene transcription and T cell proliferation without TCR engagement" J. Immunol. (1998) 161: 1645-1651 und in den Verweisen dort. Dies bestätigt, daß der Antikörper erst in bispezifischer Form, d.h. nach Bindung an das Ziel-Antigen supra-agonistisch wird. In diesem Sinn ist die supra-agonistische Wirkung selektiv.

Bischof et al., "Autonomous induction of proliferation, JNK and NFalphaB activation in primary resting T cells by mobilized CD28" Eur. J. Immunol. (2000) 30(3):876-882, konnten zwar zeigen, daß Antikörper gegen CD28 von Ratten eine T-Zell Proliferation ohne zusätzliches TCR/CD3 stimulierten, jedoch wurde dieser Effekt den besonderen CD28 Antikörpern, spezifisch für immobilisiertes CD28 zur Abkürzung der TCR/CD3-unabhängigen CD28-Rekrutierung, zugeschrieben. Im übrigen beschreiben diese Arbeiten mit monospezifischen CD28-Antikörpern zwar supra-agonistische Wirkungen, aber nicht solche, die selektiv durch Bindung an ein Ziel-Antigen ausgelöst werden.

Weitere Vorteile ergeben sich aus der Beschreibung und der beigefügten Zeichnung.

Es versteht, sich, daß die vorstehend genannten- und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

Fig. 1a    eine schematische Darstellung des Antikörper-Moleküls auf genetischer Ebene;

Fig. 1b    die Aminosäuresequenz eines erfindungsgemäßen bispezifischen Antikörper-Moleküls;

Fig. 2a    die Reinigung des rekombinanten Antikörpers durch Gelfiltration;

Fig. 2b    die Fraktionen aus der Gelfiltration, aufgetrennt durch SDS-Polyacrylamid-Gelelektrophorese;

Fig. 3    die Bindungsaktivität des monomeren und dimeren rekombinanten single-chain-Antikörpers mit CD28/9.2.27-Spezifität an Jurkat-T-Zell Lymphomzellen;

Fig. 4    die Aktivierung von peripheren Blut-mononuklearen Zellen (PBMC) durch bestrahlte SKMel63-Zellen, die für drei Tage mit dem rekombinanten Antikörperdimer oder dem rekombinanten Antikörpermonomer inkubiert wurden;

Fig. 5    das Abtöten von SKMe163-Zellen nach einer Inkubation von vier Tagen mit peripheren Blut-mononuklearen Zellen (PBMC) und dem rekombinanten Antikörper als Monomer und als Dimer;

Fig. 6    die Lyse der SKMe163- und M21-Melanom-Zellen durch periphere Blut-mononukleare Zellen (PBMC), die mit SKMe163-Zellen inkubiert wurden.

Beispiel 1:

Genetische Konstruktion und Expression des einzelkettigen, bispezifischen Antikörper-Moleküls

Die monospezifischen $scF_v$-Antikörper wurden unter Verwendung des RPAS-Systems (Pharmacia Biotech, Freiburg) aus HybridomcDNA gewonnen. Die variablen Ketten $V_L$ und $V_H$ jeweils einer Spezifität sind miteinander durch einen flexiblen 15-Aminosäurelinker verbunden, der folgende Aminosäure-Sequenz aufweist:

$(Gly-Gly-Gly-Gly-Ser)_3$

Nach Herstellung der funktionellen monospezifischen $scF_v$-Fragmente wurden diese durch einen 19-Aminosäurenlinker verbunden. Dieser Linker L entspricht einem Teil des N-Terminus der $C_H1$-Domäne des humanen IgG. Die Aminosäuresequenz des Linkers L lautet wie folgt:

Ala-Ser-Thr-Lys-Gly-Pro-Ser-Val-Phe-Pro-Leu-Ala-Pro-Ser-Ser-Ser-Gly-Ser-Gly.

Auf diese Weise wurde ein Konstrukt generiert mit der folgenden 5'→3'-Orientierung:

$(V_L-FL-V_H)_{9.3}$-L-$(V_H-FL-V_L)_{9.2.27}$-6his.

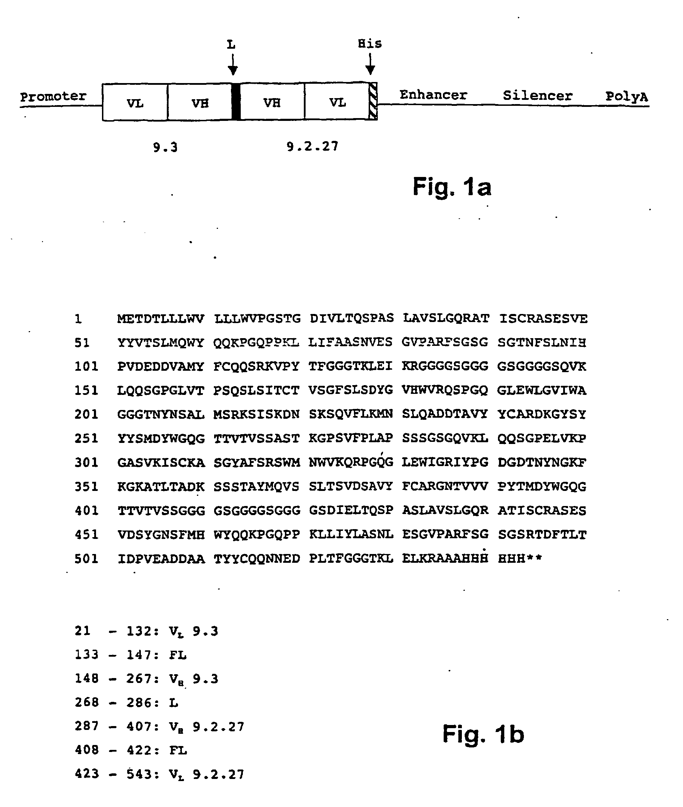

$V_L$ bezeichnet dabei jeweils die variable Domäne der leichten Kette, $V_H$ die variable Domäne der schweren Kette. Das "9.3" Antikörper-Fragment ist spezifisch für CD28, das "9.2.27" Antikörper-Fragment für Melanom-assoziierts Proteoglykan. Die Aminosäuresequenzen der Antikörper-Fragmente sind in Fig. 1b gezeigt. Der leichten Kette $V_L$ 9.3 ist eine Leader-Sequenz vorgeschaltet zur Expression des Antikörper-Moleküls in den Kulturüberstand. "L" bezeichnet den Peptid-Linker, der die beiden Spezifitäten verbindet, "FL" stellt den 15 Aminosäure langen flexiblen Linker jeweils zwischen der schweren und der leichten Kette einer Spezifität dar. Mit "6his" ist ein Aminosäurerest mit sechs Histidinen angegeben, der auch als His-Tag bezeichnet wird.

Als regulatorische Elemente wurden zum einen an das 5'-Ende der kodierenden Region ein 1,1 kB κ-Promotorfragment fusioniert, am 3'-Ende ein 5,5 kB μ-Intron, das ein Enhancer-Fragment ("Enhancer") beinhaltet, und ein 1,8 kB langer PolyA-Schwanz ("PolyA") wurde angefügt (siehe Fig. 1a). Dieses Konstrukt wurde in einen Vektor kloniert, der durch Fusion des pCDNA-3 (Stratagene, La Jolla, CA) und des pCR-Scriptvektors (Invitrogen, Groningen, The Netherlands) hergestellt wurde. Figur 1a zeigt schematisch die genetische Organisation des Antikörper-Moleküls (nicht maßstabsgetreu).

Anschließend wurden zwei Mausmyelomzellinien, P3X63Ag8 und J558, durch Elektroporation mit dem Konstrukt transfiziert. Die Produktionsraten, die mit der J558-Zellinie erreicht wurden, waren größer als die der anderen Zellinien, weshalb diese für die Reinigung des rekombinanten Antikörpers verwendet wurde.

Statt der hier beschriebenen spontanen Dimerisierung des ersten bispezifischen Antikörper-Moleküls bei seiner rekombinaten Herstellung kann die Dimerisierung auch gezielt erfolgen. Es ist ferner möglich, durch rekombinante Technologie zweite erfindungsgemäße Antikörper-Moleküle herzustellen, die bivalent für CD28 und mono- oder bivalent für ein Tumor-Antigen sind und wie die Dimere aus den ersten erfindungsgemäßen Antikörper-Molekülen effektiv T-Zellen stimulieren.

Beispiel 2:

Reinigung des rekombinanten Antikörpers

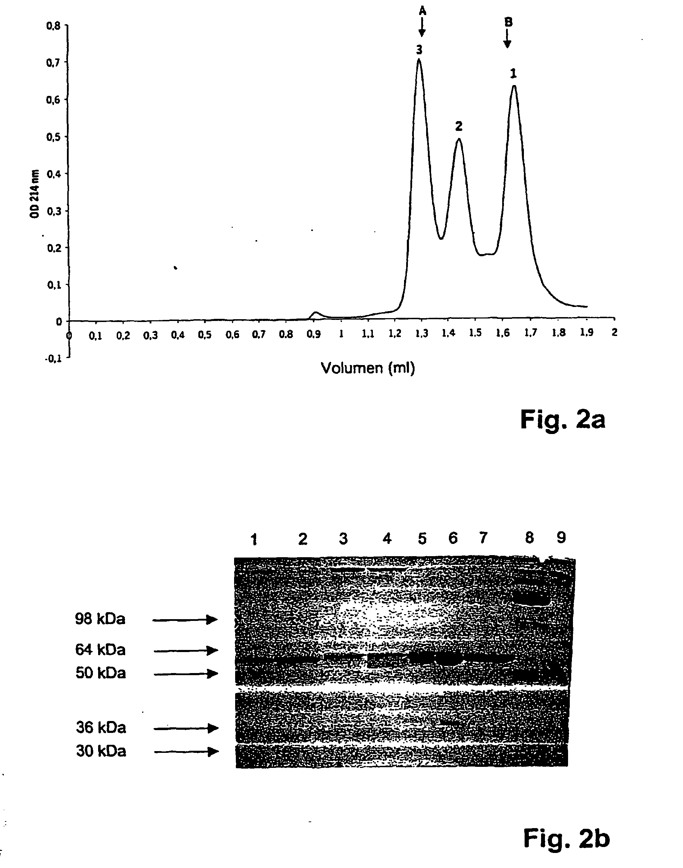

In einem ersten Reinigungsschritt wurde der Amoniumsulfatgefällte Überstand transfizierter Zellen auf eine Protein-L-Säule gegeben. Protein-L bindet bestimmte Vκ-Subtypen der leichten Ketten der Maus. Während der Gelfiltration konnten drei bestimmte Peaks identifiziert werden, die einem Molekulargewicht von ungefähr 55.000 (Peak 1), 100.000 (Peak 2) und 160.000 (Peak 3) Dalton entsprachen (siehe Fig. 2a). Die Gelfiltration wurde auf Superdex S200 Säulen (Pharmacia, Freiburg, Deutschland) durchgeführt, entweder mit konventioneller FPLC-Ausrüstung für eine präparative Trennung, oder mit dem SMART System (Pharmacia) für eine analytische Gelfiltration.

Nach einer SDS-Polyacralamid-Gelelektrophorese (SDS-PAGE) hingegen konnten nur zwei Banden beobachtet werden, die einem Molekulargewicht von ungefähr 55.000 und 160.000 Dalton entsprachen, wie in Fig. 2b dargestellt: In Spur 6 ist der Ammoniumsulfat-gefällte Überstand transfizierter Zellen vor der Gelfiltration aufgetragen. In Spur 1 ist das Peak-1-Material nach Gelfiltration aufgetragen. In Spur 2 ist Peak-2-Material nach der Gelfiltration und nach Protein-G Adsorption gezeigt, in Spur 3 Peak-2-Material nach Gelfiltration und vor Protein-G Adsorption. Die Molekulargewichtsanalysen wurden durch eine 10% SDS-PAGE durchgeführt. Es wurde Protein-G und Protein-L Material der Firma Devitron (Castrop-Rauxel, Deutschland) verwendet.

In Anbetracht dessen, daß die 160 Kilodalton-schwere Bande durch die Adsorption an Protein-G entfernt werden konnte, konnte diese Bande als Immunglobulin identifiziert werden, das durch die transfizierte Zellinie J558 selber produziert wurde.

Das gereinigte Material aus Peak 2 der Zellinien J558 und P3X63Ag8 war von dem Peak-1-Material nach SDS-PAGE nicht zu unterscheiden: beide in den zwei Peaks enthaltenen Antikörper-Spezies, besaßen ein Molekulargewicht von ungefähr 58 Kilodalton, das in Übereinstimmung mit dem erwarteten Molekulargewicht des monomeren bispezifischen single-chain-Moleküls war.

Darüber hinaus erwiesen sich die beiden Antikörper-Spezies als identisch in der N-terminalen Proteinsequenzierung. Die Protein-Sequenz-Analysen wurden mittels eines Hewlett Packard Protein Sequenzer G241 (Hewlett Packard, Waldbronn, Germany) durch Edman-Abbau durchgeführt.

Zusammenfassend konnte also gezeigt werden, daß durch Gelfiltration eine Antikörper-Spezies mit einem Molekulargewicht von ungefähr 115 Kilodalton gereinigt werden konnte (Peak-2-Material) und daß dieser Antikörper in der SDS-PAGE mit genau der Hälfte des ursprünglichen Molekulargewichts erschien, daß er jedoch die gleichen Sequenzen wie das erwartete monomere bispezifischen single-chain-Molekül aufwies. Aufgrund dessen konnte gezeigt werden, daß das Peak-2-Material Homodimere der rekombinanten bispezifischen single-chain-Moleküle (rM28-Dimer) aufweist. Das Dimer ist durch SDS-PAGE nicht nachweisbar, da es bei der Gelelektrophorese in seine Monomere zerfällt.

Beispiel 3:

Bindungsaktivitäten der verschiedenen bispezifischen Antikörperspezies

Um die Bindung der bispezifischen Antikörper zu bestimmen, wurden Jurkat- und M21-Zellen, die CD28 und das Melanom-assoziierte Proteoglykan exprimieren, mit Antikörpern verschiedener Konzentrationen inkubiert, gewaschen und mit einem anti-Histidin monoklonalen Antikörper (dia 900, Dianova, Hamburg, Deutschland) und einem Ziegen- (anti-Maus) Antikörper (Dianova) gefärbt. Die Zellen wurden im FACSCalibur mit der CellQuest Software (Becton Dickinson, San Jose, USA) analysiert.

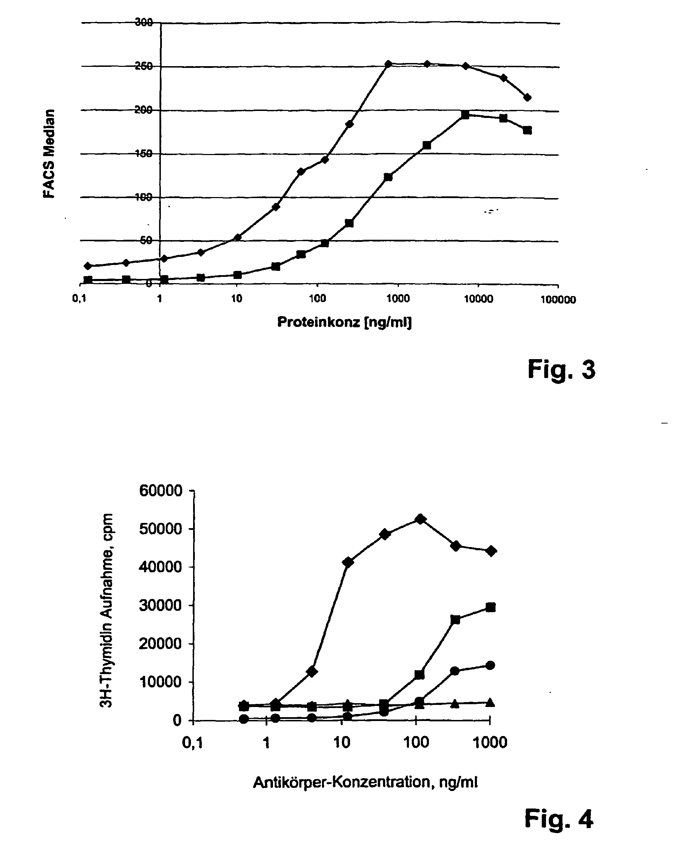

In Figur 3 ist die Bindungsaktivität der unterschiedlichen bispezifischen Antikörperspezies an Jurkat-Zellen dargestellt. Eine Bindung wurde sowohl für das Melanom-assoziierte Proteoglykan als auch für CD28 gemessen, beide Spezifitäten werden auf M21- und Jurkat-T-Zellen exprimiert. Wie aus Figur 3 zu erkennen ist, erwies sich das Bindungsverhalten des dimeren rekombinanten Antikörpers (♦ Dimer) als verbessert im Vergleich zu dem des monomeren single-chain-Antikörpers (■ Monomer).

Als Vergleich wurden außerdem chemisch hybridisierte bispezifische Antikörper (cM28) hergestellt: Antikörper, die entweder Melanom (9.2.27)-Spezifität oder CD28 (9.3)-Spezifität besaßen, wurden durch Protein A Säulenchromatographie aus Hybridomüberständen isoliert. Die Antikörper wurden fragmentiert und durch selektive Reduktion und Re-Oxidation von Disulfidbrücken der Hinge-Region hybridisiert. Das Verfahren zur chemischen Hybridisierung ist z.B. beschrieben von Jung et al.: "Target cell induced T cell activation with bi- and trispecific antibody fragments" Eur. J. Immunol. (1991) 21:2431-2435. Durch die verwendeten Reaktionsbedingungen konnte die Bildung von Homodimeren vermieden werden. Die so chemisch hybridisierten bispezifischen Antikörper zeigten in den Bindungsassays eine ähnliche Bindungsaffinität wie der monomere Antikörper (Daten nicht dargestellt).

Beispiel 4:

Targetzellinduzierte T-Zell-Aktivierung

Um Targetzell-induzierte T-Zell-Aktivierung zu messen, wurden sowohl die monomeren als auch die dimeren bispezifischen Tumor/CD28-Antikörper dreifach auf 96-well-Mikrotiterplatten mit bestrahlten (120 Gy) SKMe163-Zellen ($10^4$/well) und peripheren Blut-mononuklearen Zellen (im folgenden: PBMC) von gesunden Spendern ($10^5$/well) inkubiert. Während der letzten 18 Stunden eines 4-tägigen Inkubationszeitraumes wurde $^3$H-Thymidin hinzugefügt (0,5 $\mu$Ci/well). Die Zellen wurden geerntet und die Radioaktivität in einem Szintillationszähler (MicroBeta, Wallac) bestimmt.

In Fig. 4 ist die Induktion der T-Zell-Proliferation durch die unterschiedlichen bispezifischen Antikörperkonstrukte in Anwesenheit von SKMe163-Melanomzellen dargestellt, an die die Antikörper spezifisch binden: das chemisch hybridisierte F(ab')$_2$-Fragment (▲ cM28) erwies sich selbst bei einer hohen Antikörperkonzentration als mäßig effektiv. Im Gegensatz dazu induzierten die rekombinanten dimeren Moleküle (♦ rM28-Dimer) eine Zellaktivierung, die vergleichbar zur Stimulierung mit Phythämagglutinin (PHA) ist, schon bei Konzentrationen von 10 ng/ml. Das Monomer (■ rM28-Monomer) zeigte eine bestimmte Aktivität bei hohen Konzentrationen, wobei diese Aktivität mit der Aufbewahrungszeit stieg. Die T-Zell-Aktivierung durch das rM28-Dimer in Abwesenheit von SKMe163-Targetzellen ist durch die Kurve mit dem Symbol ● gezeigt. Bei zwei von sechs unabhängigen Versuchen zeigte cM28 eine geringe Aktivität bei Konzentrationen von >300 ng/ml. Mit diesen Ausnahmen ist das in Fig. 4 dargestellte Experiment repräsentativ.

Wenn anfänglich monomeres Material nach einer Aufbewahrungzeit von zwei Wochen bei 4°C mittels Gelfiltration wieder analysiert wurde, zeigte sich, daß mehr als 15 % des Materials in dimerer Form vorlag. Deshalb kann zumindest ein Teil der Monomer-Aktivität auf die Tatsache zurückgeführt werden, daß geringe Mengen an kontaminierendem Dimer entweder schon zu Beginn des Assays vorhanden waren oder aber erst spontan während des Verlaufs gebildet wurden.

In einigen Experimenten zeigte das dimere Molekül, nicht aber das monomere (rM28-Monomer) oder das bispezifische F(ab')$_2$-Fragment (cM28), eine gewisse Hintergrundaktivität, d.h. Induktion der T-Zell-Aktivierung ohne anwesende Targetzellen. Diese Aktivität war aber immer bedeutend niedriger als diejenige, die in Anwesenheit von Melanomzellen beobachtet wurde, und die Aktivität trat immer erst bei einer hohen Antikörperkonzentration auf. Wenn als Kontrolle UvGG eingesetzt wurde, eine Proteoglykan-negative "ovarian cancer" Karzinomzellinie, erfolgte die T-Zell-Aktivierung nicht über diesen Hintergrundlevel hinaus.

Auch bei den antigennegativen Zellen T98G und U373 konnte keine über diesen Hintergrundlevel hinausgehende T-Zell-Aktivierung festgestellt werden.

Beispiel 5 :

Abtötung von Tumorzellen

Zur Bestimmung der Abtötung von Tumorzellen wurden lebensfähige Melanomzellen dreifach in 96-well Mikrotiterplatten (5x10$^3$/well) zusammen mit PBMC und Antikörpern inkubiert. Nach vier Tagen wurden die PBMC entfernt und die Anzahl der restlichen lebensfähigen, adhärierenden Tumorzellen nach Anfärbung mit dem Tetrazolium-Salz WST (Roche Diagnostics, Mannheim, Deutschland) bestimmt. Die optische Dichte wurde in einem ELISA-Reader (Spectra Max 340, Molecular devices, Sunnyvale, CA) bestimmt und der Prozentsatz an abgetöteten Zellen berechnet.

Alternativ wurde die Cytotoxizität der PBMC nach Inkubation von lebensfähigen Tumor-Zellen in Kulturflaschen (3x10$^4$/ml) mit PBMC (6x10$^5$/ml) gemessen. Nach drei Tagen wurden die Zellen, die in Anwesenheit von SKW63-Zellen stimuliert wurden, geerntet und in einem $^{51}$Cr-Freisetzungs-Assay getestet, mit stimulierenden SKW63-Zellen und M21-Zellen als Targets. Abschließend wurden beide Zellinien mit $^{51}$Cr (40 $\mu$Ci/ml) eine Stunde lang markiert, auf 96-well-Mikrotiterplatten ausgesät (5x10$^3$/well) und mit PBMC's inkubiert, die in Anwesenheit von SKW63 und bispezifischen Antikörpern mit einem Effektor:Target-Verhältnis 30:1 stimuliert wurden. $^{51}$Cr-Abgabe wurde nach vier Stunden gemessen. Der Prozentsatz an abgetöteten Tumor-Zellen wurde nach der folgenden Standard-Formel berechnet:

$$(cpm_x - cpm_{spont}) / (cpm_{max} - cpm_{spont})$$

Dabei bezeichnet cpm$_{max}$ die Radioaktivität, die von mit Detergens behandelten Targetzellen freigesetzt wird, und cpm$_{spont}$ die spontane Freisetzung in Abwesenheit von PBMC und Antikörpern.

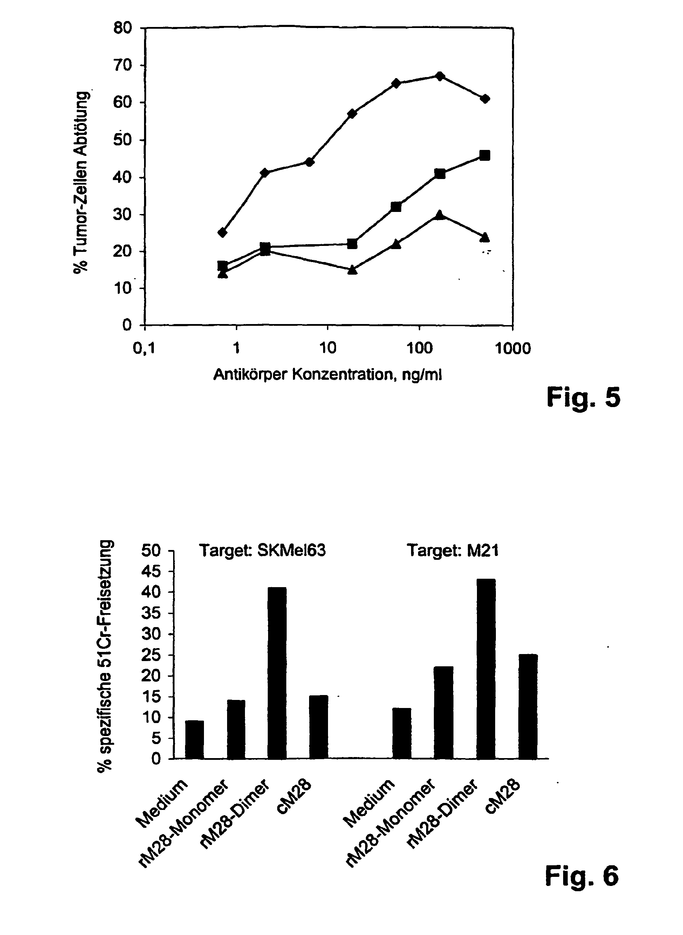

In Fig. 5 ist gezeigt, daß T-Zell-Aktivierung zu einer effektiven Abtötung von Tumorzellen führt. Nach vier Tagen wurden Melanomzellen, die mit PBMC in Anwesenheit des dimeren bispezifischen Antikörpers inkubiert wurden, beinahe vollständig abgetötet (♦ rM28-Dimer). Ähnlich wie beim Proliferationsassay war die Abtötung von Tumorzellen bedeutend verstärkt, wenn statt des monomeren (■ rM28-Monomer) der dimere (♦ rM28-Dimer) rekombinante bispezifische Antikörper verwendet wurde. Die Aktivität des chemisch hybridisierten bispezifischen Antikörpers (▲ cM28) war wiederum nur gering.

Fig. 6 zeigt die lytische Aktivität von PBMC, die für drei Tage mit SKHe163 inkubiert wurden, wobei die stimulierenden Zellen und die M21-Zellen in einem standardisierten $^{51}$Cr-Abgabeassay als Target dienten. Es ist offensichtlich, daß der dimere rekombinante Antikörper bei der Induzierung der Zelllyse am effektivsten ist.

Trotzdem scheint diese Aktivität unspezifisch zu sein, da nicht nur die stimulierenden Zellen, sondern auch eine Melanomzelle mit unterschiedlichem HLA-Typ abgetötet wurde. Deshalb ist die lytische Aktivität, die unter diesen Bedingungen gemessen wurde, nicht auf alloreaktive T-Zellen zurückzuführen.

Zusammenfassend läßt sich also sagen, daß ein Dimer aus erfindungsgemäßen ersten bispezifischen Antikörper-Molekülen mit CD28/Tumor-Spezifität ein erfolgversprechendes Mittel ist, um T-Zellen bezüglich eines bestimmten Tumors effektiv zu aktivieren, ohne daß eine zusätzliche Stimulierung des TCR/CD3-Komplexes notwendig ist.

Es ist aber nicht zwingend erforderlich, daß das Antikörper-Molekül auch für das Tumor-Antigen bivalent ist, wichtig ist die Bivalenz für CD28.

**Patentansprüche**

1. Rekombinantes bispezifisches Antikörper-Molekül mit einer ersten Bindungsstelle für den T-Zell-Rezeptor CD28 und einer zweiten Bindungsstelle für ein tumorassoziiertes Zelloberflächenantigen, wobei die variablen Domänen der jeweiligen schweren Kette ($V_H$) der beiden Bindungsstellen miteinander über einen Peptidlinker verbunden sind, der die Aminosäuresequenz Ala-Ser-Thr-Lys-Gly-Pro-Ser-Val-Phe-Pro-Leu-Ala-Pro-Ser-Ser-Ser-Gly-Ser-Gly (SEQ ID NO: 2) enthält.

2. Antikörper-Molekül gemäß Anspruch 1, wobei das tumorassoziierte Zelloberilächenantigen aus der Gruppe ausgewählt wird, die aus melanomassoziiertem Proteoglykan, Her-2/neu und CD20 besteht.

3. Antikörper-Molekül gemäß Anspruch 2, wobei das tumorassoziierte Zelloberflächenantigen melanomassoziiertes Proteoglykan ist.

4. Antikörper-Molekül gemäß einem der Ansprüche 1 bis 3, das die Sequenz wie in Fig. 1b offenbar SEQ ID NO: 3) aufweist.

5. Nukleinsäuremolekül, das ein Antikörper-Molekül gemäß einem der Ansprüche 1 bis 4 kodiert.

6. Nukleinsäuremolekül gemäß Anspruch 5, das in einem Vektor enthalten ist.

7. Zelle, die ein Nukleinsäuremolekül gemäß Anspruch 5 oder 6 enthält.

8. Zelle nach Anspruch 7, die aus der Gruppe ausgewählt wird, die aus Säugerzellen, Bakterienzellen, Insektenzellen, Pflanzenzellen und Hefenzellen besteht.

9. Verfahren zur Herstellung eines Antikörper-Moleküls gemäß einem der Ansprüche 1 bis 4, das umfasst:

   (a) Klonieren eines Nukleinsäuremoleküls, das ein solches Antikörperfragment kodiert, in einen geeigneten Vektor,
   (b) Einbringen des in (a) erhaltenen rekombinanten Vektors in eine geeignete Wirtszelle und Expression des Nukleinsäuremoleküls in dieser geeigneten Wirtszelle, und
   (c) Reinigung des in (b) erhaltenen rekombinanten Antikörper-Moleküls.

10. Pharmazeutische Zusammensetzung, die ein Antikörper-Molekül gemäß einem der Ansprüche 1 bis 4 und einen pharmazeutisch annehmbaren Träger enthält.

11. Verfahren zur selektiven Abtötung von Tumorzellen *in vitro,* das umfasst: Inkubation eines Antikörper-Moleküls gemäß einem der Ansprüche 1 bis 4, welches Spezifität für ein Zelloberflächenantigen der abzutötenden Tumorzellen aufweist, zusammen mit T-Zellen sowie diesen Tumorzellen, wobei das Verfahren **dadurch gekennzeichnet ist, dass** die alleinige Gegenwart des Antikörper-Moleküls zur tumorzellinduzierten Aktivierung der T-Zellen und in der Folge zur Abtötung der Tumorzellen ausreicht.

12. Verwendung eines Antikörper-Moleküls gemäß einem der Ansprüche 1 bis 4 zur Herstellung eines Arzneimittels zur Prophylaxe oder Therapie von Tumorerkrankungen.

**Claims**

1. A recombinant bispecific antibody molecule with a first binding site for the CD28 T-cell receptor and a second binding site for a tumor-associated cell-surface antigen, wherein the variable domains of the respective heavy chain ($V_H$) of the two binding sites are joined together via a peptide linker that contains the amino acid sequence Ala-Ser-Thr-Lys-Gly-Pro-Ser-Val-Phe-Pro-Leu-Ala-Pro-Ser-Ser-Ser-Gly-Ser-Gly (SEQ ID NO: 2).

2. The antibody molecule as claimed in claim 1, wherein the tumor-associated cell-surface antigen is selected from the group comprising melanoma-associated proteoglycan, HER-2/new and CD20.

3. The antibody molecule as claimed in claim 2, wherein the tumor-associated cell-surface antigen is melanoma-associated proteoglycan.

4. The antibody molecule as claimed in one of the claims 1 to 3, which comprises the sequence as disclosed in Fig. 1b (SEQ ID NO: 3).

5. A nucleic acid molecule that encodes an antibody molecule as claimed in one of the claims 1 to 4.

6. The nucleic acid molecule as claimed in claim 5, which is contained in a vector.

7. A cell that contains a nucleic acid molecule as claimed in claim 5 or 6.

8. The cell as claimed in claim 7, which is selected from the group comprising mammalian cells, bacterial cells, insect cells, plant cells and yeast cells.

9. A method of production of an antibody molecule as claimed in one of the claims 1 to 4, comprising:

(a) Cloning a nucleic acid molecule that encodes the said antibody fragment, in a suitable vector,
(b) Insertion of the recombinant vector obtained in (a) into a suitable host cell and expression of the nucleic acid molecule in said suitable host cell, and
(c) Purification of the recombinant antibody molecule obtained in (b).

10. A pharmaceutical composition that contains an antibody molecule as claimed in one of the claims 1 to 4 and a pharmaceutically acceptable carrier.

11. A method for selective destruction of tumor cells in vitro, comprising: Incubation of an antibody molecule as claimed in one of the claims 1 to 4, which has specificity for a cell-surface antigen of the tumor cells that are to be destroyed, together with T cells and these tumor cells, the method being **characterized in that** just the presence of the antibody molecule is sufficient for tumor-cell-induced activation of the T cells and subsequently for destruction of the tumor cells.

12. The use of an antibody molecule as claimed in one of claims 1 to 4 in the manufacture of a medicament for the prophylaxis or therapy of tumor diseases.

**Revendications**

1. Molécule d'anticorps bispécifique recombinant comportant un premier site de liaison pour le récepteur de cellule T CD28 et un second site de liaison pour un antigène de surface cellulaire associé à une tumeur, les domaines variables de la chaîne lourde (V$_H$) respective des deux sites de liaison étant reliés entre eux par un segment de liaison peptidique qui contient la séquence d'aminoacides Ala-Ser-Thr-Lys-Gly-Pro-Ser-Val-Phe-Pro-Leu-Ala-Pro-Ser-Ser-Ser-Gly-Ser-Gly- (SEQ ID n° 2).

2. Molécule d'anticorps selon la revendication 1, dans laquelle l'antigène de surface cellulaire associé à une tumeur est choisi dans le groupe qui est constitué par un protéoglycane associé à un mélanome, Her-2/neu et CD20.

3. Molécule d'anticorps selon la revendication 2, dans laquelle l'antigène de surface cellulaire associé à une tumeur est un protéoglycane associé à un mélanome.

4. Molécule d'anticorps selon l'une quelconque des revendications 1 à 3, qui a la séquence représentée sur la figure 1b (SEQ ID n° 3).

5. Molécule d'acide nucléique qui code une molécule d'anticorps selon l'une quelconque des revendications 1 à 4.

6. Molécule d'acide nucléique selon la revendication 5, qui est contenue dans un vecteur.

7. Cellule qui contient une molécule d'acide nucléique selon la revendication 5 ou 6.

8. Cellule selon la revendication 7, qui est choisie dans le groupe qui est constitué par des cellules de mammifères des cellules bactériennes, des cellules d'insecte, des cellules végétales et des cellules de levure.

9. Procédé pour la production d'une molécule d'anticorps selon l'une quelconque des revendications 1 à 4, qui comprend:

(a) le clonage d'une molécule d'acide nucléique codant un tel fragment d'anticorps, dans un vecteur approprié,
(b) l'introduction dans une cellule hôte appropriée du vecteur recombinant obtenu en (a) et l'expression de la molécule d'acide nucléique dans cette cellule hôte appropriée, et
(c) la purification de la molécule d'anticorps recombinant obtenue en (b).

10. Composition pharmaceutique contenant une molécule d'anticorps selon l'une quelconque des revendications 1 à 4 et un véhicule pharmaceutiquement acceptable.

11. Procédé pour tuer sélectivement des cellules tumorales in vitro, comprenant : l'incubation d'une molécule d'anticorps selon l'une quelconque des revendications 1 à 4, qui présente une spécificité pour un antigène de surface cellulaire des cellules tumorales à tuer, conjointement avec des cellules T ainsi que ces cellules tumorales, le procédé étant **caractérisé en ce que** la seule présence de la molécule d'anticorps suffit pour l'activation des cellules T induite par les cellules tumorales et par conséquent pour tuer les cellules tumorales.

12. Utilisation d'une molécule d'anticorps selon l'une quelconque des revendications 1 à 4, pour la fabrication d'un médicament destiné à la prophylaxie ou à la thérapie de maladies tumorales.

L     His

| Promoter | VL | VH | ▮ | VH | VL | ▨ | Enhancer | Silencer | PolyA |
|---|---|---|---|---|---|---|---|---|---|

9.3     9.2.27

## Fig. 1a

```
1    METDTLLLWV LLLWVPGSTG DIVLTQSPAS LAVSLGQRAT ISCRASESVE
51   YYVTSLMQWY QQKPGQPPKL LIFAASNVES GVPARFSGSG SGTNFSLNIH
101  PVDEDDVAMY FCQQSRKVPY TFGGGTKLEI KRGGGGSGGG GSGGGGSQVK
151  LQQSGPGLVT PSQSLSITCT VSGFSLSDYG VHWVRQSPGQ GLEWLGVIWA
201  GGGTNYNSAL MSRKSISKDN SKSQVFLKMN SLQADDTAVY YCARDKGYSY
251  YYSMDYWGQG TTVTVSSAST KGPSVFPLAP SSSGSGQVKL QQSGPELVKP
301  GASVKISCKA SGYAFSRSWM NWVKQRPGQG LEWIGRIYPG DGDTNYNGKF
351  KGKATLTADK SSSTAYMQVS SLTSVDSAVY FCARGNTVVV PYTMDYWGQG
401  TTVTVSSGGG GSGGGGSGGG GSDIELTQSP ASLAVSLGQR ATISCRASES
451  VDSYGNSFMH WYQQKPGQPP KLLIYLASNL ESGVPARFSG SGSRTDFTLT
501  IDPVEADDAA TYYCQQNNED PLTFGGGTKL ELKRAAAHHH HHH**
```

21 – 132: V$_L$ 9.3
133 – 147: FL
148 – 267: V$_B$ 9.3
268 – 286: L
287 – 407: V$_B$ 9.2.27
408 – 422: FL
423 – 543: V$_L$ 9.2.27

## Fig. 1b

Fig. 2a

Fig. 2b

**Fig. 3**

**Fig. 4**

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0212545 W

**In der Beschreibung aufgeführte Nicht-Patentliteratur**

- **TOMLINSON ; HOLLIGER.** Methods for Generating Multivalent and Bispecific Antibody Fragments. *Methods in Enzymology,* 2000, vol. 326, 461-479
- **KIPRIYANOV et al.** Bispecific Tandem Diabody for Tumor Therapy with improved Antigen Binding and Pharmacokinetics. *J. Mol. Biol.,* 1999, vol. 293, 41-56
- **TIBBEN et al.** Pharmacogenetics, biodistribution and biological effects of intravenously administered bispecific monoclonal antibody OC/TR Fab2 in ovarian carcinoma patients. *Int. J. Cancer,* 1996, vol. 66, 447-483
- **JUNG, G. ; MÜLLER-EBERHARD, H.J.** An in vitro model for tumor immunotherapy with antibody heteroconjugates. *Immunology Today,* 1988, vol. 9, 257-260
- **GROßE-HOVEST, L. et al.** Tumor growth inhibition with bispecific antibody fragments in a syngeneic mouse melanoma model: The role of targeted T cell costimulation via CD28. *Int. J. Cancer,* 1999, vol. 80, 138-144
- **HAYDEN et al.** Costimulation by CD28sFv expressed on the tumor cell surface or as a soluble bispecific molecule targeted to the L6 carcinoma antigen. *Tissue Antigen,* 1996, vol. 48, 242-254
- **ARUFFO A. et al.** *Proc. Natl. Acad. Sci. U.S.A.,* 1987, vol. 84, 8573-8577
- **SPRIEL et al.** Immunotherapeutic Perspective for Bispecific Antibodies. *Immunol. Today,* 2000, vol. 21 (8), 391-397
- **SCHOONJANS, R. et al.** Fab Chains as an Efficient Heterodimerization Scaffold for the Production of Recombinant Bispecific and Tri-specific Antibody Derivatives. *J. Immunol.,* 2000, vol. 165 (12), 7050-7057
- **SIEFKEN et al.** A CD28 associated signaling pathway leading to cytokine gene transcription and T cell proliferation without TCR engagement. *J. Immunol.,* 1998, vol. 161, 1645-1651
- **BISCHOF et al.** Autonomous induction of proliferation, JNK and NFalphaB activation in primary resting T cells by mobilized CD28. *Eur. J. Immunol.,* 2000, vol. 30 (3), 876-882
- **JUNG et al.** Target cell induced T cell activation with bi- and trispecific antibody fragments. *Eur. J. Immunol.,* 1991, vol. 21, 2431-2435